# EUROPÄISCHE PATENTANMELDUNG

(11) **EP 1 916 610 A1**
(43) Veröffentlichungstag der Anmeldung: **30.04.2008**
(21) Anmeldenummer: 07118427.9
(22) Anmeldetag: 15.10.2007
(51) Int. Cl.: G06F 19/00

(54) **Tragbare personalisierte Begleitkarte, insbesondere Patientenbegleitkarte, Informationssystem und Verfahren zur Ausgabe und/oder Weitergabe von personenbezogenen Daten**

(30) Priorität: 25.10.2006 DE 102006050350
(71) Anmelder: Siemens Aktiengesellschaft, 80333 Munich (DE)
(72) Erfinder: Kagermeier, Robert, 90427, Nürnberg (DE); Engel, Thomas, 91054, Erlangen (DE); Hassel, Jörg, 91058, Erlangen (DE); Roelofs, Frank, 91207, Lauf (DE); Weiß, Roland, 91058, Erlangen (DE)

(57) **Zusammenfassung**

Zur einfachen und schnellen Ausgabe und/oder Weitergabe von personenbezogenen Daten ist eine tragbare personalisierte Begleitkarte (2) vorgesehen, insbesondere eine Patientenbegleitkarte. Die Begleitkarte (2) umfasst eine Steuereinheit (12), einen Speicher (4) zum Speichern von personenbezogenen Daten, eine Energieversorgungseinheit (6) und eine Elektrochrom-Anzeige (10) zum Anzeige der personenbezogenen Daten.

## Beschreibung

Die Erfindung betrifft eine tragbare personalisierte Begleitkarte, insbesondere eine Patientenbegleitkarte. Die Erfindung betrifft weiterhin ein Informationssystem, insbesondere zum Einsatz in einem Krankenhaus, umfassend eine Anzahl an derartigen Begleitkarten sowie ein Verfahren zur Ausgabe und/oder Weitergabe von personenbezogenen Daten.

Zur Optimierung eines Arbeitsablaufs ist häufig ein Informationsmedium notwendig, mit dessen Hilfe Daten, insbesondere personenspezifische Daten einfach und schnell ausgegeben werden können. Zum Beispiel ist es in einem Krankenhaus zur Beschleunigung und Optimierung der Untersuchung oder der Behandlung eines Patienten von großem Vorteil, patientennah eine Patientenakte oder Begleitkarte zur Verfügung zu stellen, aus der die behandelnden Ärzte bzw. das Pflegepersonal Patientendaten, Medikationen, durchzuführende Untersuchungen, etc. ersehen können.

In einem Krankhaus werden patientenspezifische Daten üblicherweise in einer papierbasierenden oder elektronischen Patientenakte hinterlegt. Nachteiligerweise besteht bei einer Patientenakte in Papierform die Gefahr eines Verlustes und kann nicht immer patientennah zur Verfügung gestellt werden, da sie meistens in einem Archiv gelagert werden. Darüber hinaus ist bei großen Datenmengen die Auffindbarkeit von Informationen besonders schlecht. Die elektronischen Patientenakten basieren auf stationären oder tragbaren Computersystemen, welche zwar einen schnellen und auch patientennahen Zugriff auf die Patientendaten gewähren, aber die Verwendung eines kompletten, teuren, und mechanisch sehr empfindlichen Gerätes bedingen. Zudem haben diese Geräte einen sehr hohen Energiebedarf und sind deshalb nur für wenige Stunden ohne Nachladen mobil einsetzbar.

Der Erfindung liegt die Aufgabe zugrunde, eine kostengünstige, einfache und schnelle Ausgabe von personenbezogenen Daten anzugeben.

Die Aufgabe wird erfindungsgemäß gelöst durch eine tragbare personalisierte Begleitkarte, insbesondere Patientenbegleitkarte, umfassend eine Steuereinheit, einen Speicher zum Speichern von personenbezogenen Daten, eine Energieversorgungseinheit und eine Anzeige auf Basis eines digitalen Papiers, insbesondere eine Elektrochrom-Anzeige, zum Anzeigen der personenbezogenen Daten.

Eine kostengünstige und robuste Realisierung einer tragbaren Begleitkarte von nicht zu großen Dimensionen ist Dank dem Einsatz der Technologie des so genannten digitalen Papiers möglich. Unter digitalem Papier werden allgemein energiesparende Anzeigen mit folienartigem Charakter vorstanden. Einen Überblick über unterschiedliche Technologien für das digitale Papier gibt der Artikel "Digitales Papier" aus der Zeitschrift "c't - Magazin für Computer Technik", 21/2006, S. 228ff. Unter elektrochrome Anzeige sind hierbei jegliche Arten von Displays, insbesondere farbige Displays zu verstehen, die durch das Anlegen einer Gleichspannung ihre Farbe verändern. Die elektrochrome Anzeige der Begleitkarte ist sehr dünn, biegsam, leicht und relativ günstig in der Herstellung. Darüber hinaus ist eine elektrochrome Anzeige in allen Größen und Formen herstellbar, und zeichnet sich durch einen hohen Kontrast aus. Des Weiteren bietet eine Elektrochrom-Anzeige den Vorteil, dass die angezeigten Informationen über einen relativ langen Zeitraum energielos bzw. mit nur sehr geringer Leistungsaufnahme aufrechterhalten werden können und dadurch der Energiebedarf äußerst gering ist. Auf der Begleitkarte werden auf einem Speicher personenbezogene Daten gespeichert, die mittels einer Steuereinheit auf der Elektrochrom-Anzeige angezeigt werden. Die personenbezogenen Daten können im Einfachsten Fall Informationen zur Identifizierung der Person, wie z.B. Namen, Geburtsdatum oder eine betriebsinterne Identifikationsnummer enthalten. Bei einer Begleitkarte mit ausführlichen Daten, die z.B. in einem Krankenhaus als eine tragbare Patientenbegleitkarte eingesetzt wird, können außerdem Untersuchungs- und/oder Behandlungsdaten wie etwa Medikationen oder Untersuchungstermine hinterlegt werden. Die Elektrochrom-Anzeige der Patientenbegleitkarte weist beispielsweise eine Größe in Format DIN A5 bis A4 auf, so dass der Patienten sie ohne großen Aufwand während seines Krankenhausaufenthalts mit sich tragen kann. Ärzte und Pflegepersonal können somit schnell einen ersten Überblick über die Patientendaten und die wesentliche Krankengeschichte erhalten, ohne dass dafür ein zusätzliches Gerät erforderlich ist.

Gemäß einer bevorzugten Ausgestaltung umfasst die Begleitkarte weiterhin eine Kommunikationsschnittstelle. Unter Kommunikationsschnittstelle sind die Elektronikkomponenten der Begleitkarte zu verstehen, mit deren Hilfe eine Übertragung von Daten zwischen der Begleitkarte und weiteren Vorrichtungen stattfindet. Dank dieser Kommunikationsschnittstelle können nicht nur auf dem Speicher hinterlegte Daten angezeigt werden, sondern es findet auch eine Datenübertragung zwischen der Begleitkarte und weiteren Datengeräten statt, so dass die hinterlegten Informationen regelmäßig aktualisiert werden können.

Gemäß einer besonders bevorzugten Ausgestaltung ist ein RFID-Transponder vorgesehen, der die Kommunikationsschnittstelle umfasst. Insbesondere ist hierbei auch der in der Begleitkarte enthaltene Speicher ein Bauelement eines RFID-Chips. Aufgrund seiner kleinen Abmessungen kann ein derartiger RFID-Chip in die Patientenbegleitkarte integriert werden, ohne dass sich die dabei die Dimensionierung der Patientenbegleitkarte wesentlich verändert. Ein RFID-Transponder bietet eine Reihe von Vorteilen an, wie z.B. eine hohe Speicherkapazität, eine geringe Störanfälligkeit sowie die Möglichkeit einer schnellen Aktualisierung und Ergänzung der auf dem Chip hinterlegten Daten. Neben den Patientendaten können auf dem RFID-Chip zusätzlich noch Daten, wie z.B. Gebäude-Übersichten, Wegbeschreibungen, Speisepläne, Öffnungszeiten etc. mit abgelegt sein. Somit dient die Patientenbegleitkarte nicht nur zur Identifizierung des Patienten, sondern sie kann zusätzlich für den Patienten z.B. bei der Navigation im Krankenhausgebäude von Nutzen sein.

Für detaillierte Angaben zur Diagnose, Therapie etc. ist die Begleitkarte bevorzugt zum Anschluss an einen Netzwerkadapter für eine Kommunikation, insbesondere eine drahtlose Kommunikation mit einem Informationssystem ausgebildet. Beim Ankoppeln der Patientenbegleitkarte mit einem solchen externen Adapter kann eine Echtzeit-Kommunikation mit dem rechnerbasierten Informationssystem des Krankenhauses via WLAN oder Ethernet erfolgen. Es werden somit umfangreiche Daten verfügbar gemacht und eine Synchronisierung der lokal gespeicherten Daten auf der Begleitkarte ermöglicht. Der Netzwerkadapter kann entweder pro Krankenstation oder Untersuchungszimmer zur Verfügung gestellt werden, oder vom Arzt bzw. Pflegepersonal als kleines robustes tragbares Gerät eingesetzt werden.

Bei großen Krankenhäusern Kliniken oder anderen Einrichtungen ist es für die Patienten oft schwer, sich zu orientieren. Deshalb ist vorzugsweise die Kommunikationsschnittstelle dafür eingerichtet, Positionsdaten zu empfangen und die Steuereinheit dafür eingerichtet, die Positionsdaten anzuzeigen, wodurch der Patient jederzeit seine Position im Krankenhaus bestimmen und den kürzesten Weg finden kann.

Das Volumen der Patientenbegleitkarte kann besonders effektiv klein gehalten werden, indem die Steuereinheit bevorzugt eine Polymerelektronik umfasst. Bei einer Polymerelektronik werden elektrisch leitende Polymere zum Aufbau von polytronischen Anwendungen (z.B. RFID-Tags, UV-Filter, Aktoren, Sensoren, Solarzellen, etc.) verwendet. Anders als bei Molekularelektronik wird die Information nicht in einzelnen Molekülen, sondern in verschiedenen dotierten Volumina verarbeitet. Durch eine Polymerelektronik können preiswert integrierte Schaltungen im unteren Leistungsbereich auf flexiblen Substraten hergestellt werden. Eine Polymerelektronik zeichnet sich durch ihre niedrigen Herstellungskosten und ihre hohe Flexibilität aus.

Eine besonders raumsparende Ausbildung der Energieversorgungseinheit liegt vor, wenn sie bevorzugt nach Art einer Folienbatterie ausgebildet ist, wodurch sie einen besonders flachen Aufbau aufweist und sich leicht in der Begleitkarte integrieren lässt. Unter Folienbatterien sind insbesondere die vom Fraunhofer-Institut für Siliziumtechnologie ISIT entwickelten Festelektrolyt-Akkumulatoren zu verstehen, die u.a. im Fraunhofer-Magazin 2.2000 beschrieben sind.

Weiterhin bevorzugt umfasst die Energieversorgungseinheit Solarzellen, die auf dem Fotovoltaikeffekt basieren. Die fotovoltaischen Solarzellen weisen ein großes Leistungsspektrum auf und können effizient Licht im Inneren von Gebäuden zur Stromversorgung der Begleitkarte benutzen.

Vorteilhafterweise ist eine weitere Dateneingabeeinheit vorgesehen, die insbesondere nach Art einer Tastatur ausgebildet ist. Die Tastatur kann beispielsweise eine begrenzte Anzahl an Tasten aufweisen, mit deren Hilfe die Eingabe und das Aufrufen von Informationen oder die Navigation auf der e-lektrochromen Anzeige möglich ist.

Für eine bessere Übersicht der auf dem Speicher abgelegten Daten und Informationen ist die Steuereinheit vorzugsweise dafür eingerichtet, die Elektrochrom-Anzeige zum Anzeigen von mehreren Menüs anzusteuern. Hierbei kann eine große Menge an Daten klassifiziert und in Form von unterschiedlichen Menüs nach Aufruf angezeigt werden. Zum Beispiel können die Patientendaten, die Medikationsdaten oder Untersuchungstermine in einzelnen Menüs gefasst werden.

Die Begleitkarte kann eine zusätzliche Funktion aufweisen, indem die Steuereinheit gemäß einer bevorzugten Variante dafür eingerichtet ist, eine Terminerinnerung, insbesondere in Form von optischen oder akustischen Signalen, auszugeben.

Weiterhin sind gemäß einer weiteren bevorzugten Variante Maßnahmen zum Datenschutz vorgesehen, indem die Steuereinheit dafür eingerichtet ist, zumindest einen Teil der Personaldaten lediglich nach Eingabe einer ID-Kennung anzuzeigen. Die ID-Kennung oder PIN-Nummer wird vom Patienten über die Tastatur der Begleitkarte eingegeben, damit die entsprechenden Daten nur vom Patienten selbst ausgelesen werden können. Für die Freischaltung der geschützten Daten gegenüber weiteren autorisierten Personen, wie z.B. Ärzten oder Pflegepersonal, kann ein Lesegerät zum Lesen des RFID-Transponders oder ein Netzwerkadapter verwendet werden, über den die Daten in das Informationssystem des Krankenhauses übertragen werden.

Das Einsatzgebiet der Begleitkarte wird weiterhin erweitert, indem sie vorzugsweise als personenbezogene Daten sowohl Daten über die Person als auch Daten für die Person enthält. Die Daten über die Person bzw. den Patienten umfassen z.B. Name und/oder Alter sowie alle Daten in Bezug auf eine Untersuchung oder Behandlung dieses Patienten. Unter Informationen für die Person bzw. den Patienten sind die Informationen zu verstehen, die beim Aufenthalt des Patienten im Krankenhaus für ihn von Bedeutung sein können, wie z.B. eine Wegbeschreibung, Speisepläne oder sonstige Klinikinformationen.

Die Aufgabe der Erfindung wird weiterhin durch ein Informationssystem gelöst, das insbesondere zum Einsatz in einem Krankenhaus vorgesehen ist und eine Anzahl der beschriebenen Begleitkarten umfasst. Hierbei umfasst das Informationssystem mehrere Rechner, die in einem Netzwerk angeschlossen sind. Im Netzwerk können außerdem medizinische Untersuchungs- und/oder Behandlungsgeräte angeschlossen sein, die eine computerbasierte Steuerung aufweisen. Zudem umfasst das Informationssystem eine Anzahl an personalisierten Begleitkarten, die jeweils den Patienten im Krankenhaus zugeordnet sind. Jede dieser Begleitkarten ist in der Lage, über den Netzwerkadapter bzw. über den RFID-Transponder mit einzelnen Geräten dieses Informationssystems oder mit dem gesamten Informationssystem zu kommunizieren und Daten auszutauschen.

Das Informationssystem ist bevorzugt zum automatischen Auslesen, insbesondere über den RFID-Transponder, der auf der Begleitkarte hinterlegten Daten ausgebildet. Beispielsweise werden Untersuchungs- bzw. Behandlungsergebnisse des Patienten oder Daten, die für bevorstehende Untersuchungen bzw. Behandlungen erforderlich sind, von den medizinischen Geräten ausgelesen. Zudem können die personenbezogenen Daten z.B. zur Lokalisierung des Patienten benutzt werden, wenn er sich in der Reichweite eines Lese-/Sendegeräts befindet.

Die Aufgabe wird außerdem erfindungsgemäß gelöst durch ein Verfahren zur Ausgabe und/oder Weitergabe von personenbezogenen Daten mittels der beschriebenen tragbaren personalisierten Begleitkarte.

Vorteilhafterweise werden die Daten von dem Informationssystem eingelesen und weiter verarbeitet, wobei der komplette Datensatz dem Krankenpersonal schnell und automatisch zur Verfügung steht. Hierbei kann eine Untersuchung oder Behandlung durchgeführt werden, ohne dass Patientendaten manuell eingegeben oder aus unterschiedlichen Quellen zusammengestellt werden müssen.

Weiterhin von Vorteil ist, dass mittels der Begleitkarte eine Terminerinnerung, z.B. eine Medikamenteneinnahme ausgegeben wird.

Ausführungsbeispiele der Erfindung werden im Folgenden anhand einer Zeichnung näher erläutert. Hierin zeigen jeweils in schematischen, stark vereinfachten Darstellungen:
- FIG 1: die Komponenten einer Begleitkarte,
- FIG 2: eine Begleitkarte, die Patientendaten anzeigt,
- FIG 3: die Begleitkarte gemäß FIG 1, die eine Wegbeschreibung anzeigt, und
- FIG 4: ein Informationssystem eines Krankenhauses.

Gleiche Bezugszeichen haben in den verschiedenen Figuren die gleiche Bedeutung.

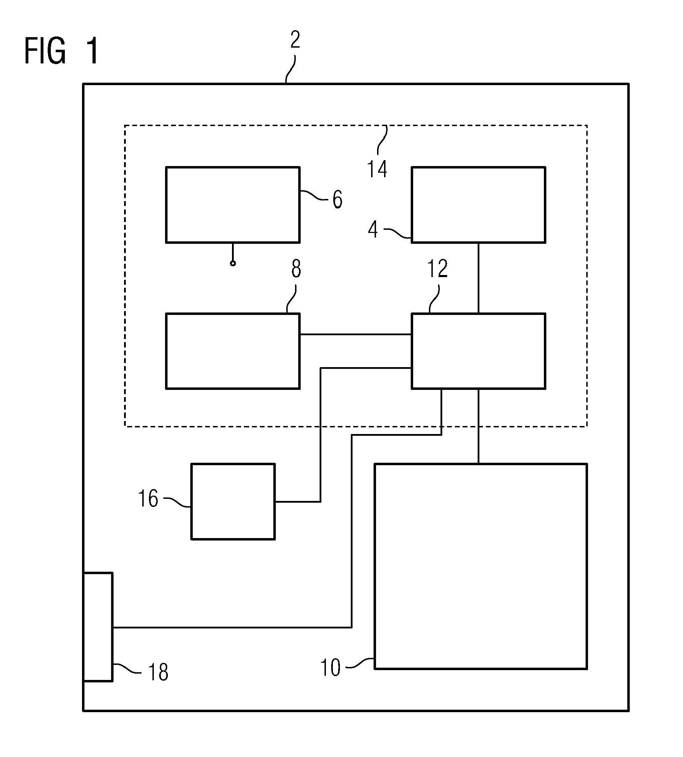

In FIG 1 ist der Elektronik-Aufbau einer tragbaren Begleitkarte 2 gezeigt. Die Begleitkarte 2 umfasst im Wesentlichen einen Speicher 4 zum Speichern von personenbezogenen Daten, eine Energieversorgungseinheit 6, die in diesem Ausführungsbeispiel nach art einer Folienbatterie ausgebildet ist, eine Sende-/Empfangeinheit 8, die eine Kommunikationsschnittstelle zur Kommunikation mit externen Geräten darstellt sowie eine Elektrochrom-Anzeige 10, auf der die personenbezogenen Daten angezeigt werden. Die Energieversorgungseinheit 6 kann außerdem durch eine hier nicht näher dargestellten fotovoltaische Solarzellen ergänzt werden, die beispielsweise auf einer Rückseite der Begleitkarte 2 angeordnet sind. Zum Ansteuern der einzelnen Komponenten der Begleitkarte 2 ist weiterhin eine Steuereinheit 12 vorgesehen.

Der Speicher 4, die Energieversorgungseinheit 6, die Sende-Empfangeinheit 8 und die Steuereinheit 12 bilden in diesem Ausführungsbeispiel einen RFID-Transponder 14, der insbesondere auf einer Polymerelektronik basiert ist. Mit Hilfe des RFID-Transponders 14 werden die personenbezogenen Daten von einem hier nicht näher dargestellten RFID-Lesegerät, das beispielsweise Teil eines Diagnostik- oder Therapiesystems ist, eingelesen und weiterverarbeitet.

Auf der Begleitkarte 2 ist eine Tastatur angebracht, die in FIG 1 symbolisch durch den Block 16 angezeigt ist und elektronisch in Verbindung mit den weiteren Komponenten der Begleitkarte, insbesondere mit der Steuereinheit 12 steht.

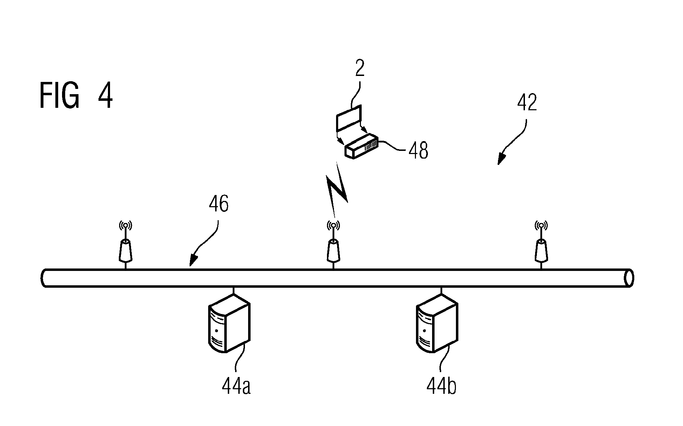

Um eine drahtlose Kommunikation der Begleitkarte mit einem Informationssystem 42 (siehe FIG 4) zu ermöglichen, ist weiterhin eine Schnittstelle 18 (z.B. ein Parallelport oder ein USB-Port) vorgesehen, an die insbesondere einen hier nicht naher gezeigten Netzwerkadapter 48 angeschlossen wird, über den der Datentransfer zwischen der Begleitkarte 2 und dem Informationssystem 42 erfolgt.

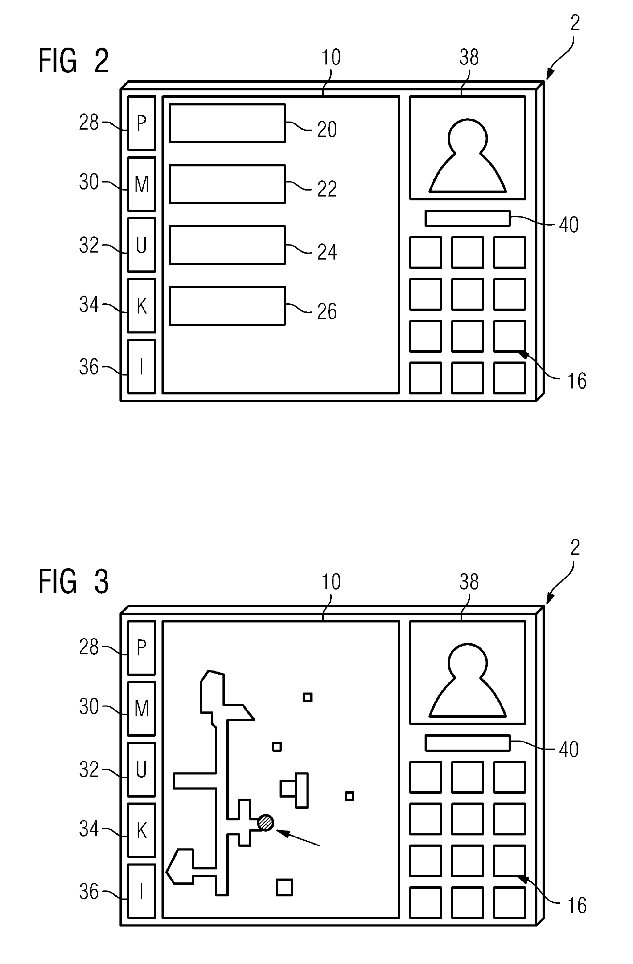

In FIG 2 ist eine Patientenbegleitkarte 2 zum Einsetzen in einem Krankenhaus oder einer anderen medizinische Einrichtung, mit einer Elektrochrom-Anzeige 10 und einer Tastatur 16 gezeigt. Die Elektrochrom-Anzeige 10 zeigt in diesem Ausführungsbeispiel ein Menüfenster, umfassend eine Reihe von vier Datenblocks 20, 22, 24, 26 mit alphanumerischen Zeichen, die die Patientendaten enthalten. Beispielsweise steht in Block 20 der Name des Patienten, in Block 22 der Vorname, in Block 24 das Geburtsdatum und in Block 26 eine krankenhausinterne Patientenidentifikationsnummer.

Das gezeigte Menüfenster wird aufgerufen durch die Betätigung einer ersten Menütaste 28 eines Menüfelds, das neben der Elektrochrom-Anzeige 10 angeordnet ist und weiterhin die Menütasten 30, 32, 34 und 36 umfasst. Durch Betätigung einer der weiteren Menütasten 30, 32, 34 und 36 ändert sich der Inhalt des Menüfensters in der Elektrochrom-Anzeige 10. In diesem Ausführungsbeispiel wird durch Betätigung der Menütaste 30 medikationsbezogene Information ausgegeben; die Menütaste 32 liefert Information über die Untersuchungstermine des Patienten; mittels der Menütaste 34 wird ein Menüfenster aufgerufen, das Kommentare und Hinweise zur Behandlung des Patienten enthält und das letzte Menüfenster, das durch die Menütaste 36 aufrufbar ist, zeigt allgemeine Informationen über das Krankenhaus an, wie z.B. einen Übersichtsplan des Krankenhauses mit der aktuellen Position des Patienten, wie es aus FIG 3 zu entnehmen ist.

In dem in FIG 2 und FIG 3 gezeigten Ausführungsbeispiel befinden sich rechts von der Elektrochrom-Anzeige 10 zwei weitere Informationsfelder 38 und 40. Zur schnellen visuellen Identifizierung des Patienten enthält das Feld 38 ein Lichtbild des Patienten und das Feld 40 die Namen des Patienten.

Die Patientenbegleitkarte 2 ist Dank der eingesetzten Technologien sehr flach ausgeführt, insbesondere mit einer Dicke von wenigen Millimetern, z.B. 1-3 mm. Die Größe der Begleitkarte 2 liegt etwa im Bereich DIN A5 bis A4. Zudem ist die Patientenbegleitkarte 2 bis zu einem gewissen Maß biegsam, robust und stoßunempfindlich. Somit kann der Patient während seines Aufenthalts im Krankenhaus die Patientenbegleitkarte 2 ohne großen Aufwand immer bei sich tragen, so dass die behandelnden Ärzte und das Pflegepersonal jederzeit schnell und einfach Zugriff zu den Patientendaten haben.

Dank des in der Begleitkarte eingebauten RFID-Transponders 14 wird der Patientendatensatz vorzugsweise von einem Lesegerät, das beispielsweise Teil eines Diagnostik- oder Therapiesystems ist, eingelesen, wodurch das Diagnostik- oder Therapiesystem den kompletten Patientendatensatz mit den zur Diagnostik oder Therapie erforderlichen Daten aus einem Informationssystem 42 des Krankenhauses abruft. Alternativ sind die therapiebezogenen Daten auf dem Speicher 4 der Begleitkarte 2 hinterlegt, so dass das RFID-Lesegerät sie einliest und direkt an das Diagnostik- oder Therapiesystem weiterleitet.

Aus datenschutzrechtlichen Gründen kann weiterhin die Eingabe einer ID-Kennung bzw. PIN-Nummer vorgesehen sein, damit die entsprechenden Daten nur von autorisierten Personen ausgelesen werden können. Für diesen Zweck kann außerdem der Speicher 4 der Begleitkarte 2 in mindestens zwei unterschiedliche Bereiche unterteilt sein, in einem verschlüsselten und einem unverschlüsselten Bereich, wobei nur bestimmten Benutzern oder Benutzergruppen der Zugang zu den Daten im verschlüsselten Bereich gewährt ist. Hierbei erfolgt die Eingabe der ID-Kennung, bzw. PIN-Nummer beispielsweise über die Tastatur 16.

Der Begleitkarte 2 gemäß den FIG 3 und FIG 3 ist außerdem eine Erinnerungsfunktion zugewiesen, indem die Begleitkarte 2 dafür ausgebildet ist, zu bestimmten eingestellten Uhrzeiten optische und/oder akustische Signale auszugeben. Der Patient kann somit z.B. an einen Termin oder eine Medikamenteneinnahme erinnert werden.

Mehrere Patientenbegleitkarten 2 sind insbesondere Teil eines rechnerbasierten Informationssystems 42 des Krankenhauses, wie es aus FIG 4 zu entnehmen ist. In dem Ausführungsbeispiel gemäß FIG 4 sind zwei Server 44a und 44b gezeigt, wobei die auf den Servern 44a, 44b abgelegten Daten über ein internes Netzwerk 46, insbesondere ein drahtloses Netzwerk (WLAN), übertragen werden. Damit eine Patientenbegleitkarte 2 mit diesem Informationssystem 42 kommunizieren kann, wird sie mit einem externen Netzwerkadapter 48 gekoppelt, welcher eine Echtzeit-Kommunikation mit den Servern 44a, 44b oder anderen Komponenten des Informationssystems 42 über das drahtlose Netzwerk 46 ermöglicht. Somit sind für den Patienten umfangreiche Informationen verfügbar, die auf den Servern 44a und 44b abgelegt sind. Außerdem kann eine Synchronisierung der auf der Begleitkarte 2 und im Informationssystem 42 gespeicherten Daten erfolgen. Das Informationssystem 42 kann neben den Servern 44a, 44b und den Begleitkarten 2 weitere computeransteuerbare Geräte umfassen, wie z.B. Diagnose- oder Therapiesysteme.

## Patentansprüche

1. Tragbare personalisierte Begleitkarte (2), insbesondere Patientenbegleitkarte, umfassend eine Steuereinheit (12), einen Speicher (4) zum Speichern von personenbezogenen Daten, eine Energieversorgungseinheit (6) und eine Anzeige (10) auf Basis eines digitalen Papiers zum Anzeigen der personenbezogenen Daten.

2. Begleitkarte (2) nach Anspruch 1, wobei die Anzeige eine Elektrochrom-Anzeige ist.

3. Begleitkarte (2) nach Anspruch 1 oder 2, die eine Kommunikationsschnittstelle (8) umfasst.

4. Begleitkarte (2) nach Anspruch 3, wobei ein RFID-Transponder (14) vorgesehen ist, der die Kommunikationsschnittstelle (8) umfasst.

5. Begleitkarte (2) nach einem der Ansprüche 3 oder 4, die zum Anschluss an einen Netzwerkadapter (48) für eine Kommunikation, insbesondere eine drahtlose Kommunikation mit einem Informationssystem (42) ausgebildet ist.

6. Begleitkarte (2) nach einem der Ansprüche 3 bis 4, wobei die Kommunikationsschnittstelle (8) dafür eingerichtet ist, Positionsdaten zu empfangen und die Steuereinheit (12) dafür eingerichtet ist, die Positionsdaten anzuzeigen.

7. Begleitkarte (2) nach einem der vorhergehenden Ansprüche, wobei die Steuereinheit (12) eine Polymerelektronik umfasst.

8. Begleitkarte (2) nach einem der vorhergehenden Ansprüche, wobei die Energieversorgungseinheit (6) nach Art einer Folienbatterie ausgebildet ist.

9. Begleitkarte (2) nach einem der vorhergehenden Ansprüche, wobei die Energieversorgungseinheit (6) Solarzellen umfasst.

10. Begleitkarte (2) nach einem der vorhergehenden Ansprüche mit einer Dateneingabeeinheit, die insbesondere nach Art einer Tastatur (16) ausgebildet ist.

11. Begleitkarte (2) nach einem der vorhergehenden Ansprüche, wobei die Steuereinheit (12) dafür eingerichtet ist, die Elektrochrom-Anzeige (10) zum Anzeigen von mehreren Menus anzusteuern.

12. Begleitkarte (2) nach einem der vorhergehenden Ansprüche, wobei die Steuereinheit (12) dafür eingerichtet ist, eine Terminerinnerung auszugeben.

13. Begleitkarte (2) nach einem der vorhergehenden Ansprüche, wobei die Steuereinheit (12) dafür eingerichtet ist, zumindest ein Teil der personalisierten Daten nach Eingabe einer ID-Kennung anzuzeigen.

14. Begleitkarte (2) nach einem der vorhergehenden Ansprüche, die Daten über die Person und Informationen für die Person enthält.

15. Informationssystem (42), insbesondere zum Einsatz in einem Krankenhaus, umfassend eine Anzahl an Begleitkarten (2) nach einem der vorhergehenden Ansprüche.

16. Informationssystem (42) nach Anspruch 15, das zum automatischen Auslesen der auf der Begleitkarte (2) hinterlegten Daten ausgebildet ist.

17. Verfahren zur Ausgabe und/oder Weitergabe von personenspezifischen Daten mittels einer tragbaren personalisierten Begleitkarte (2) nach einem der Ansprüche 1 bis 15.

18. Verfahren nach Anspruch 17, wobei die Daten von einem Informationssystem (42) eingelesen und weiterbearbeitet werden.

19. Verfahren nach einem der Ansprüche 17 oder 18, bei dem mittels der Begleitkarte (2) eine Terminerinnerung ausgegeben wird.
